# EUROPEAN PATENT APPLICATION

(11) **EP 3 673 849 A1**
(43) Date of publication of application: **01.07.2020**
(21) Application number: 19219602.0
(22) Date of filing: 24.12.2019
(51) Int. Cl.: A61B 18/14, A61M 25/00

(54) **CATHETER ELECTRODE WITH IMPROVED LATERAL HEAT TRANSFER IN ADHESIVE LAYER**

(30) Priority: 28.12.2018 US 201816235673
(71) Applicant: BIOSENSE WEBSTER (ISRAEL) LTD., 2066717 Yokneam (IL)
(72) Inventor: BEECKLER, Christopher Thomas, Irwindale, CA California 91706 (US); OKARSKI, Kevin Mark, Irwindale, CA California 91706 (US)
(74) Representative: Small, Gary James

(57) **Abstract**

An ablation electrode in a medical device includes first and second layers, and an adhesive layer. The adhesive layer, which is placed between the first and second layers, includes a mesh, which is made of a thermally conductive material defining voids in the mesh, and an adhesive that is configured to fill the voids of the mesh.

## Description

### FIELD OF THE INVENTION

The present invention relates generally to medical probes, and particularly to design and manufacturing of radiofrequency (RF) ablation catheters.

### BACKGROUND OF THE INVENTION

Catheter parts that comprise heat conductive materials including carbon, were proposed previously in literature. For example, U.S. Patent Application Publication 2016/0324575 describes an ablation device comprises an elongate body having a proximal end and a distal end, an energy delivery member positioned at the distal end of the elongate body, and a first plurality and second plurality of temperature-measurement devices carried by or positioned within the energy delivery member. The first plurality of temperature-measurement devices is thermally insulated from the energy delivery member, while the second plurality of temperature-measurement devices, which are positioned proximally to a proximal end of the energy delivery member, are thermally insulated from the energy delivery member. In some embodiments, the at least one thermal shunt member comprises a carbon-based material (e.g., Graphene, silica, etc.) with favorable thermal diffusivity properties.

As another example, Chinese Patent Application CN103194165 describes a method for preparing high-heat-conductivity conductive adhesive containing graphene. The method comprises the steps of (1) functionalizing surface of graphene, namely adding graphene to acetone solution of an organic matter containing a conjugate ring, and strongly ultrasonically agitating for 6-48 hours at 40-100 DEG C to form non-covalent modified graphene; (2) mixing epoxy resin with an epoxy diluent for 3-30 minutes at room temperature to obtain a mixture of epoxy resin and the epoxy diluent, and sequentially adding metal powder and a coupling agent to the mixture; (3) adding the non-covalent modified graphene prepared in the step (1) to the mixture in the step (2); and (4) adding a curing agent to the mixture in the step (3) to prepare the even conductive adhesive. The method has the advantages that dispersing and enhancing interface joint in an epoxy system are facilitated by functionalizing the surface of graphene by a non-covalent bond; and then graphene is mixed with metal powder to obtain the high-heat-conductivity conductive adhesive. The high-heat-conductivity conductive adhesive has the application prospect in a high-power apparatus.

### SUMMARY OF THE INVENTION

An embodiment of the present invention provides an ablation electrode in a medical device, the ablation electrode including first and second layers, and an adhesive layer. The adhesive layer, which is placed between the first and second layers, includes a mesh, which is made of a thermally conductive material comprising voids in the mesh, and an adhesive that is configured to fill the voids of the mesh.

In some embodiments, the mesh is formed from a continuous sheet by first drilling a pattern of holes to create voids.

In some embodiments, the mesh is configured to conduct heat along the ablation electrode, laterally along the adhesive layer.

In some embodiments, the material of the mesh includes graphite.

In an embodiment, the adhesive layer is predisposed over the mesh.

In another embodiment, the first and second layers are curved.

In some embodiments, the mesh has a square grid geometry.

In some embodiments, the mesh has a concentric grid geometry.

In an embodiment, the adhesive is configured to fill the voids of the mesh while the adhesive is being cured.

There is additionally provided, in accordance with an embodiment of the present invention, a method for manufacturing an ablation electrode in a medical device, the method including providing a first layer and a second layer to be adhered to one another. The first layer and the second layer are adhered using a heat conductive adhesive layer including a mesh, which is made of a thermally conductive material comprising voids, and an adhesive that is configured to fill the voids of the mesh.

The present invention will be more fully understood from the following detailed description of the embodiments thereof, taken together with the drawings in which:

### BRIEF DESCRIPTION OF THE DRAWINGS

Fig. 1 is a schematic, pictorial illustration of a system for cardiac radiofrequency (RF) ablation therapy, in accordance with an embodiment of the present invention;
Figs. 2A and 2B are schematic, pictorial illustrations of two heat conductive adhesive layers with different geometries, in accordance with embodiments of the present invention; and
Figs. 3A, 3B and 3C are schematic pictorial illustrations showing stages of a layer adhering process followed by a forming process for manufacturing a catheter tip-shell, in accordance with an embodiment of the present invention.

### DETAILED DESCRIPTION OF EMBODIMENTS

### OVERVIEW

Catheters comprising a tip electrode are commonly used for radiofrequency (RF) ablation. The tip electrode allows the ablation of tissue along a curve, where a physician repeatedly repositions the tip electrode across curved tissue. The ability of such tip electrodes to evacuate excessive heat produced by RF ablation in tissue in the immediate vicinity of the tip electrode depends on, among other factors, the heat conductivity of the tip electrode itself.

Embodiments of the present invention that are described herein provide improved ablation electrodes and methods for designing and manufacturing such electrodes. In some embodiments, an ablation electrode at the tip of a catheter (referred to herein as a tip electrode) comprises a stack of multiple layers. The different layers of which the tip electrode is made play a major role in its heat conductivity. This consideration is especially significant during cardiac ablation treatments, during which excessive heat may cause collateral damage to nearby tissue.

However, the requirement for good heat conductivity of the tip electrode sometimes contradicts the demanding adhesion processing step in tip electrode manufacture. The adhesion processing step requires adhesion by thin adhesive layers of multiple layers of dissimilar materials so as to hold the layers of the tip electrode together. This adhesion manufacturing step is particularly demanding, as the multiple layers are bent or folded to create a three-dimensional tip electrode. Unfortunately, available mechanically suitable adhesive layers do not have sufficiently high lateral thermal conductivity. As a result, such adhesive layers form a thermal barrier to the evacuation of the excessive heat described above. In the context of this disclosure the term "lateral thermal conductivity" means an ability of a layer to thermally diffuse heat in the lateral direction, i.e., in the plane of the layer itself.

In some embodiments, an ablation electrode in a medical device is provided, wherein the electrode comprises first and second layers adhered one to the other by placing an adhesive layer between the first and second layers. The disclosed adhesive layer comprises (a) a mesh made of thermally conductive material with voids in the material, and (b) an adhesive that is configured to fill the voids of the mesh.

The mesh is configured to conduct heat along the ablation electrode, laterally along the adhesive layer.

In some embodiments, the layer of adhesive is incorporated into the mesh so that the adhesive flows into the holes or voids in the mesh to provide bonding, and the high thermal conductivity material provides the lateral heat transfer. One such material is graphite mesh, which is commercially available as large sheets as thin as twenty-five microns, which has an in-plane thermal specific conductivity superior to that of metal layers of similar thickness. For instance, Panasonic PGS Graphite Sheet EYGS182303 (available from Panasonic Industrial Devices, Newark, New Jersey) is a 25 micron thick graphite sheet with an in-plane thermal conductivity of 1,600 W/(m x K), which is approximately four times greater than pure copper.

In some embodiments, the disclosed conductive mesh is initially provided as a uniform thin sheet of a heat-conductive material over which the adhesive is predisposed. Next, a grid pattern (i.e., the mesh) is formed by, for example, drilling or pressing the thin sheet. Drilling may be done by mechanical means, laser, or other suitable methods.

Later, while the adhered layers cure, the overlaid adhesive flows into the voids, securing the surfaces together with both sides of the conductive grid between them.

The disclosed technique provides a manufacturing process that can substantially improve the ability to evacuate heat from the electrode-tissue interface during an RF ablation procedure, and thereby minimize side effects, such as collateral thermal damage to nearby tissue.

### SYSTEM DESCRIPTION

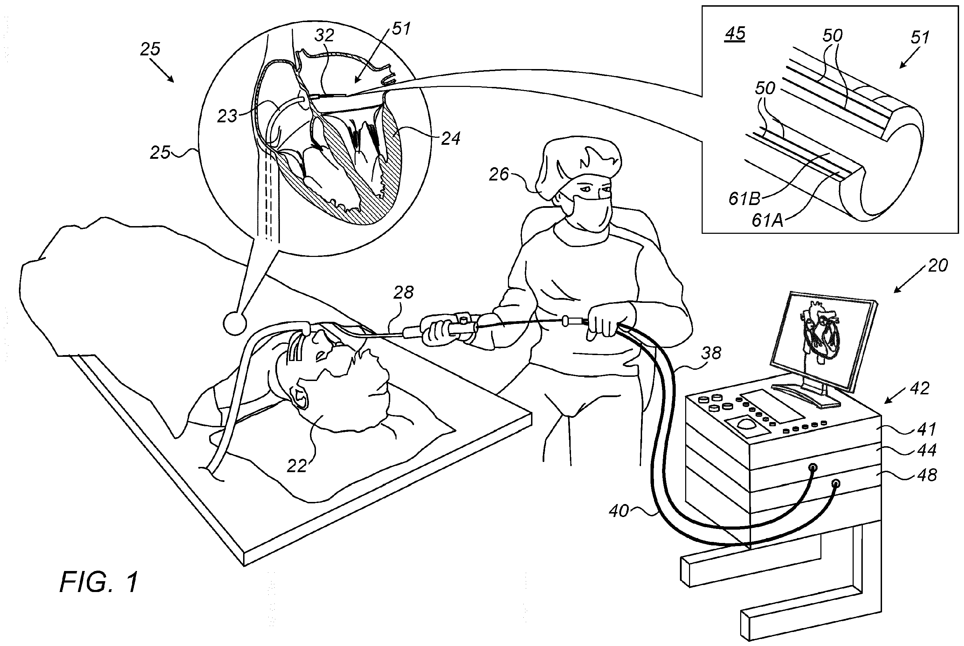

Fig. 1 is a schematic, pictorial illustration of a system 20 for cardiac radiofrequency (RF) ablation therapy, in accordance with an embodiment of the present invention. An operator 26 inserts a catheter 28 (such as a ThermoCool® catheter made by Biosense-Webster, Irvine, California) through a blood vessel into a chamber of a heart 24 of a subject 22, and manipulates the catheter so that a distal end 32 of the catheter contacts the endocardium in an area that is to be treated. A tip electrode 51 of catheter 28, seen in inset 25, comprises one or more disclosed heat conductive adhesive layers 50, seen in a schematic cross-section of electrode tip 51 in inset 45, which have high thermal conduction to enable optimal removal of heat from the treatment area. In the pictured embodiment, an adhesive layer 50 adheres together layers 61A and 61B.

After positioning distal end 32 at an ablation site, and ensuring that the tip is in contact with the endocardium, operator 26 actuates an RF energy generator 44 in a control console 42 to supply RF energy via a cable 38 to distal end 51. Meanwhile, an irrigation pump 48 supplies a cooling fluid, such as normal saline solution, via a tube 40 and a lumen in catheter 28 to the distal end. Operation of the RF energy generator and the irrigation pump may be coordinated in order to give the appropriate volume of irrigation during ablation, so as to cool the tip of the catheter and the tissue without overloading the heart with irrigation fluid. A temperature sensor in distal end 32 (not shown in the figures) may provide feedback to console 42 for use in controlling the RF energy dosage and/or irrigation volume.

Although the pictured embodiment relates specifically to the use of a tip ablation device for ablation of heart tissue, the methods described herein may alternatively be applied in other types of ablation devices, such as single-arm and multi-arm ablation devices comprising the disclosed heat conductive adhesive layers 50.

### CATHETER ELECTRODE WITH IMPROVED LATERAL HEAT TRANSFER IN ADHESIVE LAYER

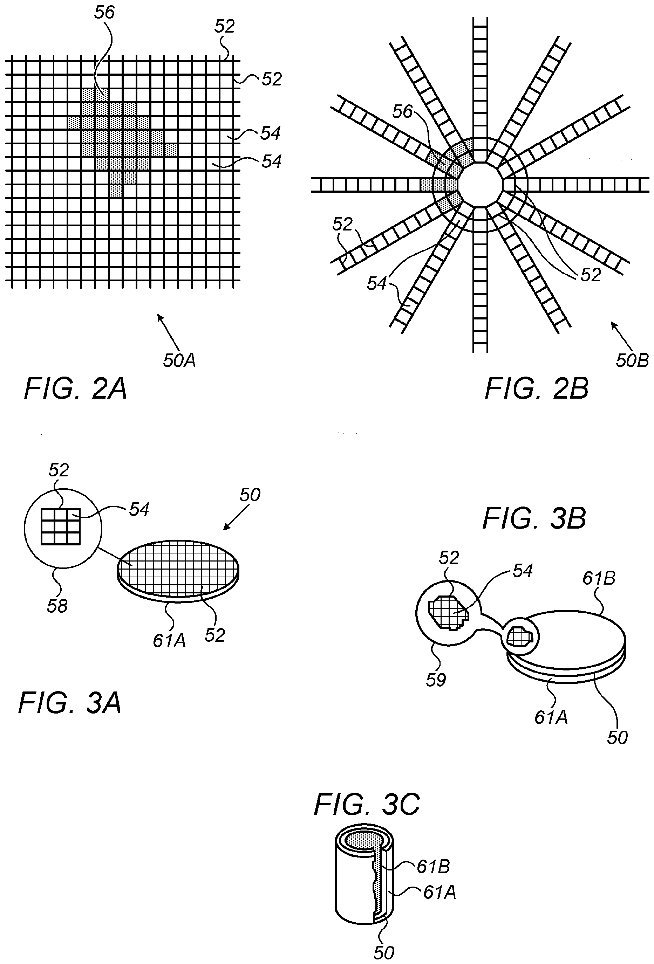

Figs. 2A and 2B are schematic, pictorial illustrations of two heat conductive adhesive layers 50A and 50B with different geometries, in accordance with embodiments of the present invention. As seen in Fig. 2A, layer 50A has a square grid geometry, while in Fig. 2B layer 50B has a concentric grid geometry. Both layers are exemplified as a mesh 52 made of strands, such as a graphite strands, which has a matrix-like structure to define voids 54, which are partially predisposed with an adhesive 56. The voids 54 are defined as the empty volume or area between the matrix-like nature of the mesh 52. In general, layers 50A and 50B are highly flexible planar-like structures, so layers 50A and 50B can be prepared in a geometry that conforms to the manufactured parts (e.g., metal flats to be bonded together and processed into a cylindrical tip-shell, as described below, but also, as another example, where the adhesive layer is prepared in a geometry that fits adhering two concentric half spheres).

In some embodiments, mesh 52 is a graphite mesh commercially available in large sheets that are as thin as 0.0004" with an in plane very-high thermal conductivity exceeding 1500 W/(mxK). Such thermal conductivity is above that of certain metal layers having high to very high thermal conductivities, in the range of 10-400 W/(mxK). In contrast, adhesives typically have a very low thermal conductivity, well below 1 W/(mxK). The disclosed technique incorporates a layer of adhesive 54 into a mesh of high thermal conductivity material 52. The mesh is flexible so that the adhesive flows into the voids 54 in the mesh to provide bonding, and the high thermal conductivity material of which mesh 52 is made provides the lateral heat transfer. In a preferred embodiment where the mesh is in sheet form, a B-staged adhesive can be used, such as, for example, Dupont® Pyralux™ LF sheet adhesive.

Figures 3A, 3B, are schematic pictorial illustrations showing stages of the layer adhering process followed by a forming process for manufacturing a catheter tip-shell, in accordance with an embodiment of the present invention.

As seen in Fig. 3A, a heat conductive mesh 52 of a heat conductive adhesive layer 50A, assumed to be already provided with an adhesive layer 54, is overlaid on a first part consisting of a flat round layer 61A. Mesh 52, with an overlaid layer of adhesive 54, is shown enlarged in inset 58.

Fig. 3B shows flat round layers 61B and 61A adhered, where a curing process (not shown) causes adhesive 54 overlaid on mesh 52 to flow into the voids, securing layers 61B and 61A adhered on both sides of the mesh 52 by a heat conductive adhesive layer 50A.

Fig. 3C shows a catheter tip-shell electrode, such as electrode tip 51, that is formed into a tubular shape from the adhered 61A and 61B planar layers by a forming process. In this forming process, planar lower layer 61A and upper layer 61B (in Fig. 3B) are deformed into a generally tubular shell (Fig. 3C) whereby the lower layer 61A defines an outer cylinder whereas the upper layer 61B defines an inner concentric cylinder, shown here in Fig. 3C. A design and manufacturing method of an irrigated tip-shell electrode using deep-drawing is described in U.S. Patent Application 15/730,223, filed October 11, 2017, entitled "Method of Producing a Densely Perforated Tip-shell From Flat Geometry," which is assigned to the assignee of the present patent application and whose disclosure is incorporated herein by reference. As disclosed by the aforementioned application, the catheter tip-shell electrode can be formed from a planar structure into a tubular structure by a deep drawing process in which a punch is pressed against a a specially perforated sheet, causing the sheet to deform into a tubular shell with perforations. Details of this process is described at pages 9 and 10 as well as Figures 3A, 3B and 3C of the aforementioned U.S. Patent Application 15/730,223, attached hereto this Appendix.

The example configuration shown in Figures 3A, 3B and 3C is chosen purely for the sake of conceptual clarity. For example, substrate layer 61A may already be curved, and the disclosed heat conductive adhesive layer and adhering methods can be applied to adhere to any curved or three-dimensional parts with surfaces to be adhered, where the parts require efficient heat conductivity by the adhering layer.

Although the embodiments described herein mainly address design and manufacturing of catheter parts, the methods described herein can also be used in other medical and non-medical applications. For example, the disclosed techniques can be used with the design and manufacturing of parts for aviation or consumer electronics.

It will thus be appreciated that the embodiments described above are cited by way of example, and that the present invention is not limited to what has been particularly shown and described hereinabove. Rather, the scope of the present invention includes both combinations and sub-combinations of the various features described hereinabove, as well as variations and modifications thereof which would occur to persons skilled in the art upon reading the foregoing description and which are not disclosed in the prior art. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

## Claims

1. An ablation electrode in a medical device, the electrode comprising:
first and second layers; and
an adhesive layer placed between the first and second layers, the adhesive layer comprising:
a mesh comprising a thermally conductive material defining voids in the mesh; and
an adhesive that is configured to fill the voids of the mesh.

2. The ablation electrode according to claim 1, wherein the mesh is configured to conduct heat along the ablation electrode, laterally along the adhesive layer.

3. The ablation electrode according to claim 1, wherein the material of the mesh comprises graphite.

4. The ablation electrode according to claim 1, wherein the adhesive layer is predisposed over the mesh.

5. The ablation electrode according to claim 1, wherein the first and second layers are curved.

6. The ablation electrode according to claim 1, wherein the mesh comprises a square grid geometry or a concentric grid geometry.

7. The ablation electrode according to claim 1, wherein the adhesive is configured to fill the voids of the mesh while the adhesive is being cured.

8. A method for manufacturing an ablation electrode in a medical device, the method comprising:
providing a first layer and a second layer to be adhered to one another; and
adhering the first layer and the second layer using a heat conductive adhesive layer comprising:
a mesh comprising a thermally conductive material defining voids in the mesh; and
an adhesive that is configured to fill the voids of the mesh.

9. The manufacturing method according to claim 8, wherein the mesh is configured to conduct heat along the ablation electrode, laterally along the adhesive layer.

10. The manufacturing method according to claim 8, wherein the material of the mesh comprises graphite.

11. The manufacturing method according to claim 8, wherein adhering the first layer and the second layer comprises predisposing the adhesive over the mesh.

12. The manufacturing method according to claim 8, wherein the first and second layers are curved.

13. The manufacturing method according to claim 8, wherein the mesh has a square grid geometry or a concentric grid geometry.

14. The manufacturing method according to claim 8, wherein adhering the first layer and the second layer comprises filling the voids with the adhesive while the adhesive is being cured.

15. The manufacturing method according to claim 8, and comprising forming the electrode into a tubular shape by deforming the adhered first and second layers from a planar shape into a tubular shape.
